# FASCICULE DE BREVET EUROPEEN

(11) **EP 1 797 201 B1**
(45) Date de publication et mention de la délivrance du brevet: **03.03.2010**
(21) Numéro de dépôt: 05809297.4
(22) Date de dépôt: 06.10.2005
(51) Int. Cl.: C12Q 1/68, C07H 21/00

(54) **AMORCES UNIVERSELLES ET LEUR UTILISATION POUR LA DETECTION ET L'IDENTIFICATION DE VEGETAUX DANS DES MELANGES COMPLEXES**
UNIVERSELLE PRIMER UND DEREN VERWENDUNG ZUR ERKENNUNG UND IDENTIFIZIERUNG VON PFLANZENMATERIALIEN IN KOMPLEXEN MISCHUNGEN
UNIVERSAL PRIMERS AND THEIR USE FOR DETECTING AND IDENTIFYING PLANT MATERIALS IN COMPLEX MIXTURES

(30) Priorité: 08.10.2004 FR 0410648
(43) Date de publication de la demande: 20.06.2007
(73) Titulaire: UNIVERSITE JOSEPH FOURIER (GRENOBLE 1), F-38041 Grenoble Cédex (FR); CENTRE NATIONAL DE LA RECHERCHE SCIENTIFIQUE, 75794 Paris Cedex 16 (FR)
(72) Inventeur: TABERLET, Pierre, F-38660 LA TERRASSE (FR); GIELLY, Ludovic, F-38320 EYBENS (FR); MIQUEL, Christian, Philippe, F-73000 CHAMBERY (FR)
(74) Mandataire: Fiorucci, Hélène
(86) Numéro de dépôt international: PCT/FR2005/002470
(87) Numéro de publication internationale: WO 2006/040448

(56) Documents cités:
- DE-A1- 10 239 585
- POINAR HENDRIK N ET AL: "Molecular coproscopy: Dung and diet of the extinct ground sloth Nothrotheriops shastensis" SCIENCE (WASHINGTON D C), vol. 281, no. 5375, 17 juillet 1998 (1998-07-17), pages 402-406, XP002328098 ISSN: 0036-8075
- TABERLET P ET AL: "UNIVERSAL PRIMERS FOR AMPLIFICATION OF THREE NON-CODING REGIONS OF CHLOROPLAST DNA" PLANT MOLECULAR BIOLOGY, vol. 17, no. 5, 1991, pages 1105-1110, XP002051822 ISSN: 0167-4412 cité dans la demande
- DEMESURE B ET AL: "A SET OF UNIVERSAL PRIMERS FOR AMPLIFICATION OF POLYMORPHIC NON-CODING REGIONS OF MITOCHONDRIAL AND CHLOROPLAST DNA IN PLANTS" MOLECULAR ECOLOGY, BLACKWELL SCIENCE, OXFORD, GB, vol. 4, no. 1, 1995, pages 129-131, XP001147382 ISSN: 0962-1083

## Description

La présente invention concerne des oligonucléotides et leur utilisation comme amorces universelles pour la détection et l'identification d'espèces végétales notamment dans des substrats complexes ou dégradés.

On connaît différentes méthodes permettant l'identification de végétaux basées sur l'analyse du génome, mais aucune ne permet pour l'instant de travailler sur des substrats dégradés et/ou complexes.

Les méthodes d'empreintes génétiques sont ainsi basées sur l'analyse du génome complet. L'objectif de ces méthodes est de fournir une empreinte génétique propre à chaque individu (identification de l'individu et non de l'espèce). Cependant, bien que ce ne soit pas l'objectif initial, elles peuvent aussi permettre dans une certaine mesure l'identification de l'espèce. Toutes ces méthodes nécessitent l'obtention d'ADN de bonne qualité (non dégradé), et sans mélange avec des ADN exogènes (provenant d'autres organismes). De ce fait, il est impossible d'utiliser ces approches pour l'identification de végétaux dans des substrats dégradés ou complexes.

A titre d'exemple de méthode d'empreinte génétique, on peut citer les méthodes AFLP et DArT.

La méthode AFLP "Amplified Fragment Length Polymorphism" est actuellement très utilisée à la fois en génétique des populations et en cartographie génétique (Vos P, Hogers R, Bleeker M, Reijans M, van de Lee T, Hornes M, Frijters A, Pot J, Peleman J, Kuiper M, Zabeau M (1995) AFLP: a new technique for DNA fingerprinting. Nucleic Acids Research, 23, 4407-4414 ; US Patent 6,045,994). Elle est basée sur une digestion/ligation de l'ADN génomique, suivie de deux amplifications successives utilisant des amorces PCR particulières de manière à simplifier le génome pour le rendre analysable par électrophorèse. Elle nécessite de l'ADN de très bonne qualité, et en quantité suffisante (plusieurs centaines de nanogrammes d'ADN en général). Il est absolument impossible d'utiliser cette approche de manière pertinente pour l'analyse de substrats dégradés et complexes.

La méthode DArT "Diversity Array Technology" est basée sur une approche très similaire (digestion/ligation puis amplification), mais en diffère par la méthode d'analyse (hybridation) (Jaccoud D, Peng K, Feinstein D, Kilian A (2001) Diversity arrays: a solid state technology for sequence information independent genotyping. Nucleic Acids Research, 29, e25 ; US patent n° 6,713,258). Il est également impossible d'utiliser cette approche sur des substrats dégradés et complexes.

D'autres méthodes sont basées sur l'amplification et le séquençage. D'un point de vue théorique, le séquençage d'une région homologue suffisamment longue (plusieurs centaines de paires de bases) possède le potentiel pour permettre l'identification de l'espèce chez les végétaux. Une telle région doit être encadrée par des zones très conservées autorisant la conception d'amorces universelles pour l'amplification. L'ADN nucléaire n'est pas très approprié, car son accès est très difficile voire impossible dans le cas de substrats dégradés Cependant, en ce qui concerne l'ADN nucléaire, les ITS (Internal Transcripted Spacer de l'ADN ribosomique) ont été utilisés pour la détection et l'identification de végétaux. Des amorces universelles ont été décrites chez les champignons et se sont avérées fonctionner également chez les végétaux (White TJ, Bruns T, Lee S, Taylor J (1990) Amplification and direct sequencing of fungal ribosomal RNA genes for phylogenetics In: PCR protocols, a guide to methods and applications (eds. Innis MA, Gelfand DH, Sninski JJ, White TJ), pp. 315-322. Academic Press, San Diego, California). De ce fait, cette région de quelques centaines de paires de bases a été utilisée pour effectuer des phylogénies entre espèces proches chez les végétaux (Baldwin BG (1992) Phylogenetic utility of the internal transcribed spacers of nuclear ribosomal DNA in plants: an example from the Compositae. Molecular Phylogenetics and Evolution, 1, 3-16 ; Gielly L, Yuan Y-M, Küpfer P, Taberlet P (1996) Phylogenetic use of noncoding regions in the genus Gentiana L.: chloroplast trnL (UAA) intron versus nuclear ribosomal internal transcribed spacer sequences. Molecular Phylogenetics and Evolution, 6, 460-466) et pour identifier les espèces (voir par exemple Linder C, Moore L, Jackson R (2000) A universal molecular method for identifying underground plant parts to species. Molecular Ecology, 9, 1549-1559 ou le site web de la société "Bioprofiles": http://www.bioprofiles.co.uk/). Cependant, cette région possède des inconvénients. Premièrement, elle est trop longue pour être utilisée dans le cas de substrats très dégradés, de plus il s'agit de séquences nucléaires certes répétées, mais de manière moindre que celles présentes dans l'ADN chloroplastique. Deuxièmement, les amorces peuvent amplifier plusieurs types de séquence au sein d'une même espèce. Enfin, les amorces ne sont pas réellement universelles et il peut être difficile d'obtenir une amplification chez certaines espèces.

En revanche, les ADN mitochondrial et chloroplastique sont présents de manière hautement répétées dans chaque cellule (plusieurs centaines de copies). Il en résulte qu'ils représentent une cible pour l'amplification beaucoup plus accessible dans le cas de substrats dégradés. L'ADN mitochondrial est cependant trop peu variable chez les végétaux.

Ainsi , plusieurs articles décrivent des amorces universelles ciblant différentes régions de l'ADN chloroplastique (Taberlet P, Gielly L, Pautou G, Bouvet J (1991) Universal primers for amplification of three non-coding regions of chloroplast DNA. Plant Molecular Biology, 17, 1105-1109 ; Demesure B, Sodzi N, Petit RJ (1995) A set of universal primers for amplification of polymorphic non-coding regions of mitochodrial and chloroplast DNA in plants. Molecular Ecology, 4, 129-131 ; Dumolin-Lapègue S, Pemonge M-H, Petit RJ (1996) An enlarged set of consensus primers for the study of organelle DNA in plants. Molecular Ecology, 5, 393-397 ; et Hamilton MB (1999) Four primer pairs for the amplification of chloroplast intergenic regions with intraspecific variation. Molecular Ecology, 8, 521-523). Certaines d'entre elles ont été largement utilisées pour amplifier et séquencer des régions variables de l'ADN chloroplastique. Il s'agit principalement des amorces c et d (Taberlet P, Gielly L, Pautou G, Bouvet J (1991) Universal primers for amplification of three non-coding regions of chloroplast DNA. Plant Molecular Biology, 17, 1105-1109) qui amplifient l'intron du gène de l'ARN de transfert pour la Leucine, codon UAA (*trn*L UAA). Actuellement, plusieurs milliers de séquences de cet intron sont disponibles dans les bases de données publiques (GenBank). L'intron du gène *trn*L (UAA) est très variable, mais possède aussi des parties conservées lié au fait qu'il peut constituer des structures secondaires (Simon D, Fewer D, Friedl T, Bhattacharya D (2003) Phylogeny and self-splicing ability of the plastid tRNA-Leu group I intron. Journal of Molecular Evolution, 57, 710-720). Cependant, ces amorces c et d amplifient des régions trop longues pour être utilisées sur des substrats dégradés.

Une autre solution pour l'identification spécifique a été proposée par Bobowski *et al*. (Bobowski B, Hole D, Wolf P, Bryant L (1999) Identification of roots of woody species using polymerase chain reaction (PCR) and restricted fragment length polymorphism (RFLP) analysis. Molecular Ecology, 8, 485-491): amplification du gène *rbc*L à l'aide d'amorces universelles, et caractérisation du produit d'amplification par digestion enzymatique suivi d'une migration sur gel (le produit pourrait être également caractérisé par séquençage direct).

Cependant, toutes ces amorces amplifient des régions trop longues pour être utilisées sur des substrats dégradés. C'est la raison pour laquelle d'autres amorces ont été définies par Poinar *et al*. (Poinar HN, Hofreiter M, Spaulding WG, Martin PS, Stankiewicz BA, Bland H, Evershed RP, Possnert G, Pääbo S (1998) Molecular coproscopy: Dung and diet of the extinct ground sloth Nothrotheriops shastensis. Science, 281, 402-406) afin d'amplifier des fragments plus courts, compatibles avec l'analyse de restes "fossiles" (coprolithes d'un paresseux disparu dans ce cas). Ces auteurs ont conçu des amorces dans le gène chloroplastique *rbc*L. Les fragments amplifiés permettent tout juste d'identifier la famille, et ces amorces ne sont pas vraiment universelles. Malgré cela, en l'absence d'alternative, Willerslev *et al*. (Willerslev E, Hansen AJ, Binladen J, Brand TB, Gilbert MTP, Shapiro B, Bunce M, Wiuf C, Gilichinsky DA, Cooper A (2003) Diverse plant and animal genetic records from Holocene and Pleistocene sediments. Science, 300, 791-795) ont utilisé les amorces de Poinar *et al.* (Poinar HN, Hofreiter M, Spaulding WG, Martin PS, Stankiewicz BA, Bland H, Evershed RP, Possnert G, Pääbo S (1998) Molecular coproscopy: Dung and diet of the extinct ground sloth Nothrotheriops shastensis. Science, 281, 402-406) dans leur analyse de l'ADN extrait du permafrost (sol gelé). Leur objectif était de caractériser les ADN végétaux encore présents dans des sols. Là aussi, seules les familles ont pu être identifiées.

Pour résumer, soit les systèmes proposés pour l'instant se basent sur des séquences trop longues pour être efficaces dans l'analyse de substrats dégradés, soit le niveau de variabilité des fragments courts n'est pas assez élevé pour être vraiment utile dans l'identification des végétaux. Les régions à la fois suffisamment courtes et suffisamment variables sont rares et aucune n'a été caractérisée.

Pour remédier aux inconvénients de l'état de la technique, la présente invention propose de nouveaux oligonucléotides et leur utilisation comme amorces universelles pour la détection et l'identification d'espèces végétales.

Les oligonucléotides de la présente invention permettent d'amplifier une région très courte mais également très variable de l'intron du gène *trnL* (UAA) de l'ADN chloroplastique.

Un premier avantage de la présente invention est que les oligonucléotides et les méthodes de la présente invention permettent la détection et l'identification des végétaux dans des substrats complexes ou dégradés tels que des substrats transformés (par la chaleur, la lyophilisation, etc.) car la région amplifiée est à la fois courte et très variable.

Un autre avantage de la présente invention est que la région amplifiée est non seulement très variable entre espèces végétales mais possède également des régions flanquantes très conservées permettant l'amplification de la région d'intérêt dans différentes espèces végétales à l'aide d'amorces universelles.

Un autre avantage de la présente invention est que l'intron du gène *trnL* (UAA) est l'une des rares séquences chloroplastiques pour laquelle plusieurs milliers de séquences sont disponibles dans les bases de données telles que GenBank (http://www.ncbi.nlm.nih.gov). L'analyse de la région variable amplifiée à l'aide des amorces universelles permet donc d'identifier l'espèce végétale correspondante en se référant aux séquences disponibles dans les bases de données.

Des méthodes permettant l'identification de végétaux dans des substrats complexes ou dégradés présentent un grand intérêt. On citera par exemple les applications dans l'industrie agroalimentaire où, par exemple, le respect des critères de traçabilité impose le développement de nouvelles méthodes d'analyses permettant d'identifier la composition détaillée en espèces végétales de préparations alimentaires.

### DESCRIPTION DE L'INVENTION

Un premier objet de la présente invention est une paire d'oligonucléotides dans laquelle le premier oligonucléotide s'hybride à la séquence de la SEQ ID No. 68 et le deuxième oligonucléotide s'hybride à la séquence de la SEQ ID No. 69 dans des conditions de stringence suffisantes pour l'amplification sélective d'une région variable de l'intron du gène chloroplastique *trn*L du tabac dont la séquence est représentée à la SEQ ID No. 3.

Un autre objet de la présente invention est une paire d'oligonucléotides dans laquelle premier oligonucléotide s'hybride à la séquence de la SEQ ID No. 68 et le deuxième oligonucléotide s'hybride à la séquence de la SEQ ID No. 69 dans des conditions de stringence suffisantes pour l'amplification sélective d'une région variable de l'intron du gène chloroplastique *trn*L des végétaux dont la séquence est représentée aux SEQ ID Nos. 24-67.

Typiquement, l'hybridation s'effectue à 55°C dans un tampon d'amplification comprenant du MgCl₂ 2mM.

Dans un mode de réalisation préféré de l'invention, le premier oligonucléotide est choisi dans le groupe comprenant les SEQ ID Nos. 1, 4-15 et le deuxième oligonucléotide est choisie dans le groupe comprenant les SEQ ID Nos. 2, 16-23.

L'invention concerne aussi des oligonucléotides dont la séquence est choisie dans le groupe comprenant la SEQ ID No. 1, la SEQ ID No. 2, les SEQ ID Nos. 4-15 et les SEQ ID Nos. 16-23.

L'invention concerne aussi des polynucléotides dont la séquence est choisie dans le groupe comprenant les SEQ ID Nos. 24-67.

Dans un mode de réalisation avantageux de l'invention, les paires d'oligonucléotides, les oligonucléotides et les polynucléotides selon l'invention sont immobilisés sur un support solide.

Un autre objet de la présente invention est un procédé d'amplification d'une région variable de l'ADN chloroplastique des végétaux comprenant les étapes suivantes :
a) on dispose d'un échantillon comprenant de l'ADN génomique végétal ;
b) on amplifie une région variable de l'ADN chloroplastique avec une paire d'oligonucléotides selon l'invention ou avec au moins un oligonucléotide selon l'invention.

Dans un mode de réalisation particulier, la région variable de l'ADN chloroplastique amplifiée à l'étape b) est un polynucléotide dont la séquence est choisie dans le groupe comprenant les SEQ ID Nos. 24-67.

Dans un autre mode de réalisation particulier, le procédé d'amplification d'une région variable de l'ADN chloroplastique des végétaux selon l'invention comprend une étape d'extraction de l'ADN chloroplastique avant l'étape d'amplification b).

De préférence, on amplifie la région variable de l'ADN chloroplastique par une réaction d'amplification en chaîne par polymérase (PCR).

L'invention concerne aussi un procédé de détection d'une espèce végétale dans un échantillon comprenant les étapes suivantes :
a) on dispose d'un échantillon suspecté de contenir une espèce végétale ;
b) on effectue une réaction d'amplification avec une paire d'oligonucléotides selon l'invention, ou avec au moins un oligonucléotide selon l'invention ;
c) on détecte l'obtention d'un produit d'amplification attestant de la présence d'une espèce végétale dans l'échantillon.

Dans un mode de réalisation particulier, le produit d'amplification est un polynucléotide dont la séquence est choisie dans le groupe comprenant les SEQ ID Nos. 24-67.

Dans un autre mode de réalisation, le procédé de détection d'une espèce végétale dans un échantillon selon l'invention comprend une étape d'extraction de l'ADN avant l'étape d'amplification b).

De préférence, on effectue une réaction d'amplification en chaîne par polymérase (PCR).

L'invention se rapporte aussi à un procédé d'identification d'une espèce végétale dans un échantillon comprenant les étapes suivantes :
a) on dispose d'un échantillon suspecté de contenir une espèce végétale ;
b) on effectue une réaction d'amplification avec une paire d'oligonucléotides selon l'invention, ou avec au moins un oligonucléotide selon l'invention ;
c) on analyse le produit d'amplification obtenu pour identifier l'espèce végétale contenue dans l'échantillon.

Dans un mode de réalisation particulier, le produit d'amplification est un polynucléotide dont la séquence est choisie dans le groupe comprenant les SEQ ID Nos. 24-67.

Dans un mode de réalisation, à l'étape c), on détermine la séquence du produit d'amplification pour identifier l'espèce végétale contenue dans l'échantillon.

Dans un autre mode de réalisation, à l'étape c), on hybride le produit d'amplification avec au moins une séquence végétale de référence pour identifier l'espèce végétale contenue dans l'échantillon.

De préférence, la séquence de référence est choisie dans le groupe comprenant les SEQ ID Nos. 3 et 24-67.

Dans un autre mode de réalisation, à l'étape c), on analyse le produit d'amplification par électrophorèse pour identifier l'espèce végétale contenue dans l'échantillon.

L'invention se rapporte aussi à l'utilisation de la région variable du gène de l'intron du gène chloroplastique *trn*L des végétaux correspondant aux positions 49425 à 49466 de l'ADN chloroplastique du tabac pour la détection et l'identification d'espèces végétales.

Dans un mode de réalisation particulier, la région variable du gène de l'intron du gène chloroplastique *trn*L des végétaux est un polynucléotide dont la séquence est choisie dans le groupe comprenant les SEQ ID Nos. 24-67.

La présente invention concerne des polynucléotides dérivés de deux régions très conservées de l'ADN chloroplastique des végétaux. Ces polynucléotides dérivés de régions dont la séquence est très conservée dans tout le règne végétal, en particulier chez les angiospermes et les gymnospermes, peuvent être utilisés comme amorces universelles pour l'amplification ou le séquençage de l'ADN de chloroplaste des végétaux.

En outre, on a trouvé que, de façon particulièrement avantageuse, les régions conservées dont sont dérivés les polynucléotides de la présente invention flanquent une région à la fois courte et très variable de l'ADN chloroplastique. La variablité de cette région entre espèces végétales peut donc être utilisée pour distinguer et identifier les espèces végétales.

Selon la présente invention, on entend par "polynucléotide " une chaine nucléotidique simple brin ou son complémentaire ou une chaine nucléotidique double brin pouvant être de type ADN ou ARN. De préférence, les polynucléotides de l'invention sont de type ADN, notamment d'ADN double brin.

Le terme "polynucléotide" désigne également les oligonucléotides et les polynucléotides modifiés. Typiquement, les polynucléotides modifiés peuvent contenir des nucléotides modifiés. Alternativement, ces polynucléotides modifiés sont des polynucléotides conjugués à des réactifs de liaison (biotine par exemple) ou à des réactifs marqués (marqueurs fluorescents par exemple). Classiquement, les réactifs de liaison ou les réactifs marqués conjugués aux polynucléotides facilitent la purification ou la détection de ces polynucléotides.

Selon l'invention, on entend par « oligonucléotide » un polynucléotide constitué d'une courte séquence de nucléotides, dont le nombre varie d'une à quelques dizaines, mais généralement inférieur à 100 bases. Le terme « polynucléotide » désigne donc aussi les oligonucléotides.

On entend par « amorce » une courte séquence d'oligonucléotides qui, hybridée avec une matrice d'acide nucléique, permet à une polymérase d'entamer la synthèse d'un nouveau brin de l'ADN. Le brin produit à partir de l'amorce est complémentaire au brin utilisé comme matrice.

Avantageusement, les polynucléotides de la présente invention peuvent être immobilisés sur un support solide. On connaît les support solides adaptés à l'immobilisation de polynucléotides ou d'oligonucléotides notamment pour la fabrication des puces à ADN. Il existe de nombreuses variétés de puces à ADN, qui se distinguent par le type de support utilisé, la nature, la densité et le mode de fixation ou de synthèse des séquences nucléotidiques sur le support, et les conditions de lecture. Ces techniques sont connues de l'homme du métier. On entend également par support solide des supports de type microsphères tels que les produits FLEXMAP™ de la société LUMINEX® et les produits LiquidChip™ de la société QIAGEN®.

De manière générale, les polynucléotides de la présente invention sont isolés ou purifiés de leur environnement naturel. De préférence, les polynucléotides de la présente invention peuvent être préparés par les techniques classiques de biologie moléculaire telles que décrites par Sambrook et al. ( Molecular Cloning : A Labratory Manual, 1989) ou par synthèse chimique.

On entend par « espèce végétale » tout organisme vivant faisant partie du règne végétal.

L'invention concerne également une paire d'oligonucléotides dans laquelle le premier oligonucléotide s'hybride à une première région très conservée de l'ADN chloroplastique et le deuxième oligonucléotide s'hybride à une deuxième région très conservée de l'ADN chloroplastique dans des conditions de stringence suffisantes pour l'amplification sélective d'une région variable de l'intron du gène chloroplastique *trn*L des végétaux. La séquence de la première région conservée correspond à la séquence de l'amorce g (SEQ ID No.1) et de sa séquence complémentaire (SEQ ID No. 68). La séquence de la deuxième région conservée correspond à la séquence de l'amorce h (SEQ ID No.2) et de sa séquence complémentaire (SEQ ID No. 69).

Chez le tabac qui peut être utilisé comme espèce végétale de référence les paires d'oligonucléotides de la présente invention permettent l'amplification sélective de la région variable de l'intron du gène chloroplastique *trn*L du tabac dont la séquence est représentée à la SEQ ID No. 3.

Les séquences des paires d'oligonucléotides de la présente invention sont choisies de telle façon que le premier oligonucléotide s'hybride à la SEQ ID No. 68 et le deuxième oligonucléotide s'hybride à la SEQ ID No. 69 dans des conditions de stringence suffisantes pour permettre l'amplification sélective de la région variable de l'intron du gène chloroplastique *trn*L des végétaux.

L'homme du métier connaît les réactions d'amplification d'ADN et les conditions de stringence permettant une amplification sélective et notamment les conditions température d'hybridation et de composition du tampon d'hybridation.

L'homme du métier pourra donc facilement définir différentes variantes des amorces g (SEQ ID No. 1) et h (SEQ ID No. 2) en utilisant des techniques de routine. Ces variantes s'hybrident aux séquences de référence et permettent l'amplification sélective de la région variable d'intérêt de l'ADN chloroplastique. Certaines variantes possibles de l'amorce g sont représentées aux SEQ ID Nos. 4-15 et certaines variantes possibles de l'amorce h sont représentées aux SEQ ID Nos. 16-23. Habituellement, les variations de séquence sont plutôt introduites à l'extrémité 5' des oligonucléotides pour ne pas compromettre la réaction d'amplification. Classiquement on peut par exemple introduire des nucléotides supplémentaires à l'extrémité 5' des oligonucléotides.

Par "s'hybrider", on entend selon l'invention les séquences qui s'hybrident avec la séquence de référence à un niveau supérieur au bruit de fond de manière significative. Le niveau du signal généré par l'interaction entre la séquence capable de s'hybrider de manière sélective et les séquences de référence est généralement 10 fois, de préférence 100 fois plus intense que celui de l'interaction des autres séquences d'ADN générant le bruit de fond. Les conditions d'hybridation stringentes permettant une hybridation sélective sont bien connues de l'homme du métier. En général la température d'hybridation et de lavage est inférieure d'au moins 5°C au Tm de la séquence de référence à un pH donné et pour une force ionique donnée. Typiquement la température d'hybridation est d'au moins 30°C pour un polynucléotide de 15 à 50 nucléotides et d'au moins 60°C pour un polynucléotide de plus de 50 nucléotides. A titre d'exemple, l'hybridation est réalisée dans le tampon suivant: 6X SSC, 50 mM Tris-HCl (pH 7.5), 1 mM EDTA, 0.02% PVP, 0.02% Ficoll, 0.02% BSA, 500 µg/ml denatured salmon sperm DNA. Les lavages sont par exemple réalisés successivement à faible stringence dans un tampon 2X SSC, 0,1%SDS, à moyenne stringence dans un tampon 0,5X SSC, 01%SDS et à forte stringence dans un tampon 0,1X SSC, 0,1 %SDS. L'hybridation peut bien entendu être effectuée selon d'autres méthodes usuelles bien connues de l'homme du métier (voir notamment Sambrook et al., Molecular Cloning : A Labratory Manual, 1989).

De préférence, les polynucléotides s'hybridant de manière sélective à un polynucléotide de référence conservent la fonction de la séquence de référence. Dans la présente invention, la fonction des polynucléotides est l'amplification d'une région variable de l'ADN chloroplastique.

Par « stringence » on entend la rigueur des conditions opératoires (notamment la température et la force ionique) dans lesquelles se déroule une hybridation moléculaire.

Par « amplification » on entend toute amplification enzymatique *in vitro* d'une séquence définie d'ADN.

Habituellement, l'amplification comporte des cycles successifs (généralement de 20 à 40) d'amplification, eux-mêmes composés de trois phases : après une étape de dénaturation (séparation des deux brins de la double hélice) de l'ADN, le positionnement des amorces (courtes séquences d'oligonucléotides spécifiquement choisies) en face de leurs séquences complémentaires, sur les brins d'ADN, et leur fixation sur ces cibles constitue la deuxième phase du procédé (hybridation). La phase d'extension fait intervenir une enzyme, l'ADN polymérase, qui synthétise, à partir des amorces, le brin complémentaire de celui ayant servi de matrice. La répétition de ce cycle conduit à l'amplification exponentielle du fragment d'ADN.

L'invention concerne aussi un procédé d'amplification d'une région variable de l'ADN chloroplastique des végétaux à l'aide des polynucléotides, des oligonucléotides et/ou des paires d'oligonucléotides selon l'invention.

Les procédés d'amplification de séquence d'ADN sont bien connues de l'homme du métier et largement décrits dans la littérature. On citera la réaction d'amplification en chaîne par polymérase (PCR) mais tout type de réaction d'amplification peut être utilisée dans les procédés selon l'invention.

Etant donné que les séquences des polynucléotides selon la présente invention sont fortement conservées dans tout le règne végétal, ces polynucléotides peuvent être utilisés pour la détection d'espèces végétales.

On entend par « détection » la détermination de la présence d'une espèce végétale dans un échantillon mais aussi la mesure et la quantification d'une espèce végétale dans un échantillon.

A l'aide des polynucléotides de la présente invention il est maintenant possible d'amplifier une région très variable de l'ADN de chloroplaste. La séquence de cette région diffère d'une espèce végétale à l'autre de telle sorte que chaque séquence est spécifique d'une espèce ou d'un petit nombre d'espèces très proches. Une fois que la région variable a été amplifiée, sa séquence est analysée pour identifier l'espèce végétale. L'analyse peut être effectuée par différentes méthodes bien connues de l'homme du métier. Il peut s'agir d'un séquencage complet ou partiel suivi d'une comparaison avec des séquences connues. Alternativement, il peut s'agir de la détermination du degré d'homologie avec des séquences connues (séquences de référence) en faisant appel à des techniques d'hybridation par exemple. Une autre possibilité est l'analyse par électrophorèse puis la comparaison avec des séquences de référence. Les procédés selon la présente invention permettent donc de déterminer l'identité d'une espèce végétale présente dans un échantillon.

On entend par « échantillon » dans une procédure d'analyse, la substance à mesurer. Dans la présente invention, l'échantillon comprend habituellement une substance organique suspectée de contenir une espèce végétale. De façon avantageuse, les procédés de la présente invention permettent l'analyse d'échantillons constitués de matière en décomposition ou dégradée par chauffage, lyophilisation, congélation ou par tout autre traitement entraînant une dégradation de l'ADN. Les procédés de la présente invention permettent ainsi la détection d'espèces végétales dans des aliments transformés par exemple. Une autre application des procédés de la présente invention est la détection d'espèces végétales dans des substrats issus de sols gelés (permafrost) ou dans des restes fossilisés.

L'échantillon peut subir un traitement avant d'effectuer la réaction d'amplification à l'aide des polynucléotides de l'invention. Typiquement, il peut s'agir d'une étape d'extraction de l'ADN selon des techniques de routine bien connues de l'homme du métier.

On entend par « extraction » l'opération consistant à extraire une substance d'un milieu à l'aide d'un solvant par exemple ou par toute autre méthode physico-chimique.

L'invention concerne aussi l'utilisation de la région variable du gène de l'intron du gène chloroplastique *trn*L des végétaux correspondant aux positions 49425 à 49466 de l'ADN chloroplastique du tabac pour la détection et l'identification d'espèces végétales.

A partir de la séquence de référence du tabac (Shinozaki K, Ohme M, Tanaka M, Wakasugi T, Hayashida N, Matsubayashi T, Zaita N, Chunwongse J, Obokata J, Yamaguchi-Sinozaki K, Ohto C, Torazawa K, Ment B, Sugita M, Deno H, Kamogashira T, Yamada K, Kusuda J, Takaiwa F, Kato A, Tohdoh N, Shimada H (1986) The complete nucleotide sequence of the tobacco chloroplast gnome. Plant Molecular Biology Reporter, 4, 110-147) et des positions de la région variable sur cette séquence de référence, l'homme du métier peut identifier les séquences correspondantes chez d'autres espèces végétales à l'aide de techniques de routine.

### DESCRIPTION DU LISTAGE DE SEQUENCES

SEQ ID No. 1 : Amorce g.

SEQ ID No. 2 : Amorce h.

SEQ ID No. 3 : Séquence variable amplifiée de *Nicotiana tabacum*.

SEQ ID No. 4-15 : Variantes de l'amorce g.

SEQ ID No. 16-23 : Variantes de l'amorce h.

SEQ ID No. 24-67 : Séquence variable amplifiée de différentes espèces végétales. SEQ ID No. 68 : Séquence de la région complémentaire à l'amorce g.

SEQ ID No. 69 : Séquence de la région complémentaire à l'amorce h.

### DESCRIPTION DES FIGURES

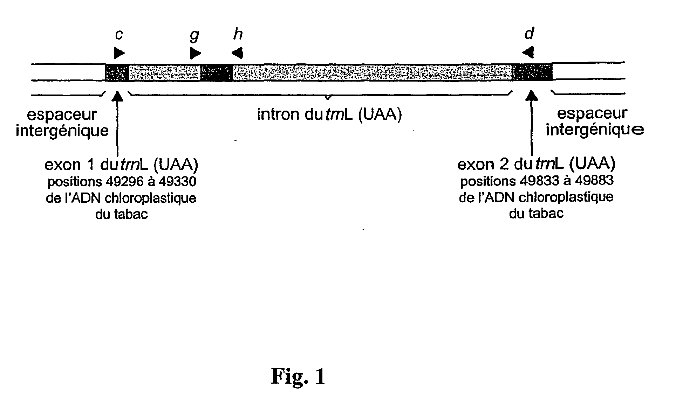

Figure 1 : Localisation de la zone étudiée et des amorces universelles sur la séquence de l'ADN chloroplastique de tabac (Shinozaki K, Ohme M, Tanaka M, Wakasugi T, Hayashida N, Matsubayashi T, Zaita N, Chunwongse J, Obokata J, Yamaguchi-Sinozaki K, Ohto C, Torazawa K, Ment B, Sugita M, Deno H, Kamogashira T, Yamada K, Kusuda J, Takaiwa F, Kato A, Tohdoh N, Shimada H (1986) The complete nucleotide sequence of the tobacco chloroplast genome. Plant Molecular Biology Reporter, 4, 110-147) ; c et d représentent les amorces définies par Taberlet et *al*. (Taberlet P, Gielly L, Pautou G, Bouvet J (1991) Universal primers for amplification of three non-coding regions of chloroplast DNA. Plant Molecular Biology, 17, 1105-1109), g et h représentent les amorces universelles définies dans le cadre de cette demande de brevet.

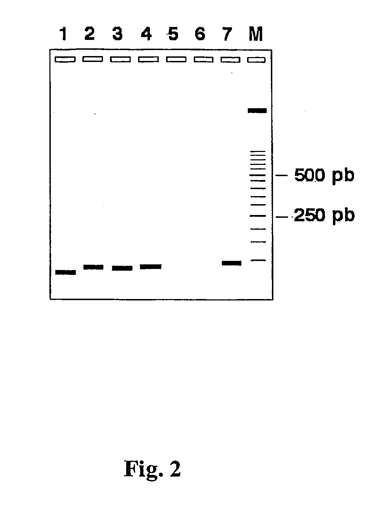

Figure 2 : Exemples d'amplifications obtenues avec les amorces g et h, à partir d'extraits d'ADN provenant de substrats dégradés. 1, pomme de terre cuite; 2, pâte cuite; 3 et 4, potage en sachet lyophilisé; 5, contrôle négatif de l'extraction (réaction d'amplification à partir d'une extraction sans subtrat); 6, contrôle négatif d'amplification (réaction d'amplification sans extrait d'ADN); 7, contrôle positif (ADN de *Cyclamen*); M, marqueur de poids moléculaire. Il est intéressant de noter que le fragment correspondant à la pomme de terre cuite (79 pb) est plus court que celui correspondant à la pâte cuite et donc au blé (92 pb).

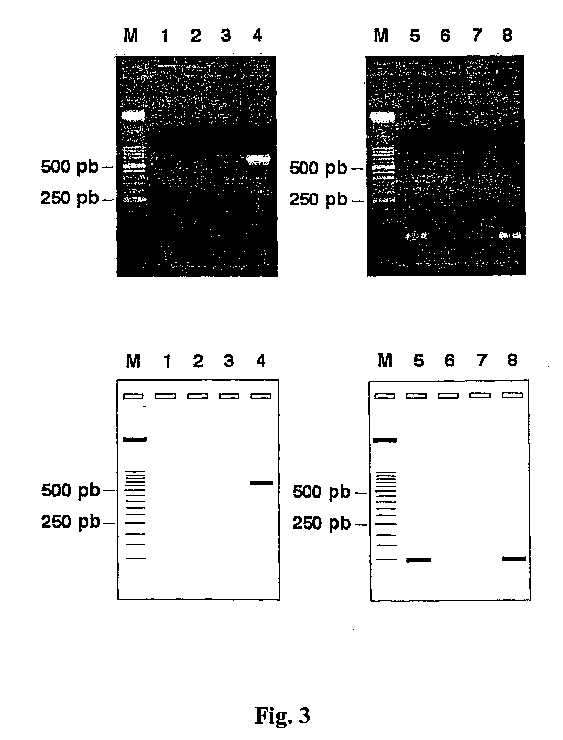

Figure 3 : Expérimentation comparant l'efficacité des amorces c-d (pistes 1-4) et g-h (pistes 5-8) pour l'amplification d'ADN extrait de substrat dégradé (mie de pain). M, marqueur de poids moléculaire. 1 et 5, ADN extrait de mie de pain. 2 et 6, contrôle d'extraction. 3 et 7, contrôle d'amplification. 4 et 8, contrôle positif.

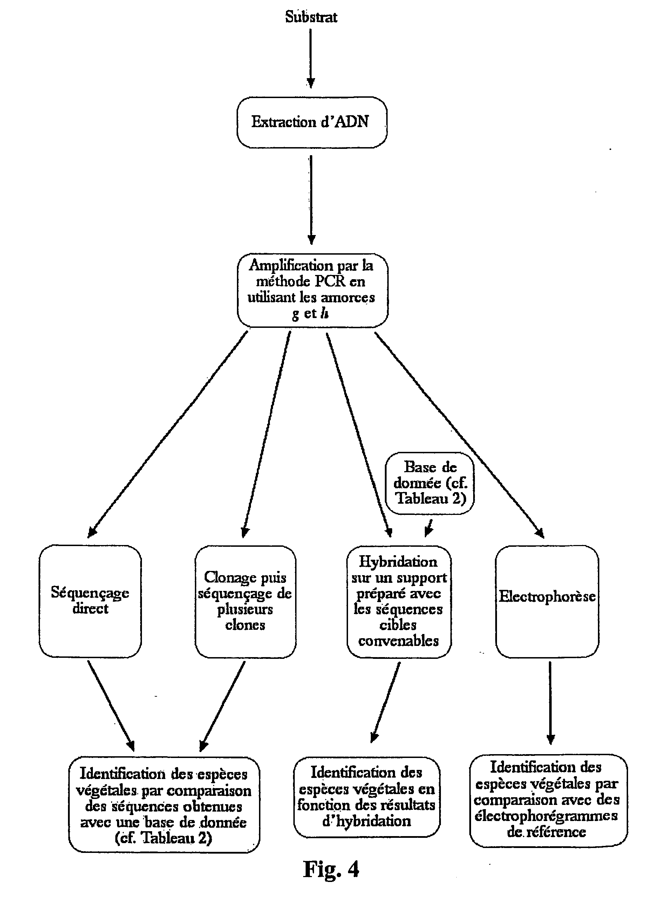

Figure 4 : La Figure 4 présente, sous forme schématique, la démarche générale qui pourrait être appliquée pour identifier des végétaux en utilisant certains modes de réalisation de l'invention. La première étape consiste à extraire l'ADN à partir du substrat. La seconde étape est l'amplification de l'extrait à l'aide de la méthode PCR (Polymerase Chain Reaction). L'analyse du produit d'amplication constitue la troisième étape. Quatre solutions alternatives se présentent. La première possibilité d'analyse ne concerne que les substrats simples (une seule espèce végétale présente) et consiste à séquencer directement le produit d'amplification avec les méthodes classiques. Les deuxièmes et troisièmes possibilités sont réservées pour les substrats complexes contenant un mélange de végétaux. L'analyse est soit effectuée après clonage du produit d'amplification puis séquençage de plusieurs clones (voir Tableau 4 pour un exemple de résultat), soit après hybridation sur un support avec les séquences cibles potentielles (méthode non illustrée, qui implique la connaissance préalable des espèces susceptibles d'être présentes). Une dernière possibilité d'analyse consiste à caractériser les produits d'amplification par électrophorèse (soit dénaturante, soit non dénaturante de type SSCP). Cette dernière possibilité peut aussi bien être utilisée pour des substrats simples (une seule espèce végétale présente) ou pour des substrats contenant un mélange.

En ce qui concerne l'analyse par séquençage direct, les conditions PCR utilisées pour la détection par électrophorèse sont les suivantes :

### EXEMPLES

### 1) Séquençage direct

En ce qui concerne l'analyse par séquençage direct, les conditions PCR utilisées pour la détection par électrophorèse sont les suivantes :
a) Conditions d'amplification pour détection avec amorce g marquée par un fluorochome :
   (i) volume final : 25µl
   (ii) MgCl2: 2 mM
   (iii) dNTP : 0,2 mM chacun
   (iv) Amorces : 1 µM chacune
   (v) Taq polymérase (Amplitaq Gold, Perkin Elmer) : 1 unité
   (vi) BSA : 0,2 µl par tube
   (vii) Volume d'extrait d'ADN utilisé : 2,5 µl
   (viii) Dénaturation initiale de 10 mn à 95°C
   (ix) Nombre de cycles : 35 (à ajuster en fonction de l'extrait)
   (x) Dénaturation : 30 s à 95°C, Hybridation : 30 s à 55°C, pas d'étape d'élongation

   Ces conditions visent à réduire l'artefact "+A" qui gène l'interprétation des résultats.
b) Conditions d'amplification pour détection avec amorce h marquée par un fluorochome :
   (i) volume final : 25µl
   (ii) MgCl2: 2 mM
   (iii) dNTP : 0,2 mM chacun
   (iv) Amorces : 1 µM chacune
   (v) Taq polymérase (Amplitaq Gold, Perkin Elmer) : 1 unité
   (vi) BSA : 0,2 µl par tube
   (vii) Volume d'extrait d'ADN utilisé : 2,5 µl
   (viii) Dénaturation initiale de 10 mn à 95°C
   (ix) Nombre de cycles : 35 (à ajuster en fonction de l'extrait)
   (x) Dénaturation : 30 s à 95°C, Hybridation : 30 s à 55°C, Elongation : 60 s à 72°C
   (xi) Elongation finale: 90 minutes à 72°C
Ces conditions visent à favoriser l'artefact "+A" pour faciliter l'interprétation des résultats.

### 2) Amorces universelles

Le tableau 1 représente les séquences des amorces universelles utilisées pour amplifier la région variable de l'ADN chloroplastique permettant d'identifier les végétaux après extraction et amplification à partir de substrats dégradés. Les positions de la base 3' sur la séquence de référence du tabac sont indiquées dans le tableau (Shinozaki K, Ohme M, Tanaka M, Wakasugi T, Hayashida N, Matsubayashi T, Zaita N, Chunwongse J, Obokata J, Yamaguchi-Sinozaki K, Ohto C, Torazawa K, Ment B, Sugita M, Deno H, Kamogashira T, Yamada K, Kusuda J, Takaiwa F, Kato A, Tohdoh N, Shimada H (1986) The complete nucleotide sequence of the tobacco chloroplast genome. Plant Molecular Biology Reporter, 4, 110-147).

**Tableau 1**

| **Amorce** | **Séquence** | **Position de la base 3' sur la séquence du tabac** |
|---|---|---|
| g | | 49425 |
| h | | 49466 |

L'alignemei : de ces deux régions flanquantes montre (Tableaux 1 et 2) qu'il est possible de définir des amorces qui sont universelles chez les végétaux supérieurs (Angiospermes et Gymnospermes). Après avoir aligné plusieurs centaines de séquences d'introns du *trn*L (UAA), les quelques variations de séquences observées au niveau de la zone où nous avons défini les amorces démontrent que les amorces sont réellement universelles (voir Tableau 2). En effet, la différence observée avec la séquence de l'amorce implique au plus un seul mésappariement n'affectant en aucun cas les trois dernières bases du côté 3' de l'amorce. De ce fait, on peut prédire avec certitude que les amorces g et h sont universelles.

### 3) Variabilité de la région amplifiée

Le Tableau 2 montre les variations de la zone amplifiée par les amorces g et h pour différentes espèces végétales entrant dans la composition des aliments (voir aussi le Tableau 3). Ces séquences ont soit été importées de GenBank (base de données publique de séquence d'ADN) soit été effectuées dans notre laboratoire.

Deux régions très conservées encadrent une partie très variable d'une longueur d'environ 20 à 100 paires de bases (Figure 1). Une telle région représente donc la cible idéale pour une identification des végétaux à partir de substrats dégradés (dans ces conditions, il est souvent difficile d'obtenir des produits d'amplification pour des fragments d'une longueur supérieure à 120 paires de bases). Nous n'avons pas trouvé d'autres régions répondant à ces critères. Il semble donc que le système que nous proposons soit unique.

Le tableau 2 représente l'alignement de séquences montrant d'une part la zone sur laquelle ont été définies les amorces universelles, et d'autre part la variabilité de la région amplifée. Les nucléotides soulignées dans les régions correspondant aux amorces indiquent les mésappariements avec les amorces universelles g et h. Concernant la région amplifiée, les soulignements indiquent des séquences identiques.

**Tableau 2**

| **Nom scientifique** | **séquence de la région correspondant à l'amorce *g*** | **séquence de la région amplifiée** | **séquence de la région correspondant à l'amorce *h*** |
|---|---|---|---|
| *Theobroa cacao* | | | |
| *Beta vulgaris* | | | |
| *Castanea sativa* | | | |
| *Cannabis sativa* | | | |
| *Cicer arietinun* | | | |
| *Saccharum officinarum* | | | |
| *Asparagus officinalis* | | | |
| *Triticum aestivum* | | | |
| *Secale cereale* | | | |
| *Oryza sativa* | | | |
| *Panicum miliaceum* | | | |
| *Ribes aureum* | | | |
| *Fragaria vesca* | | | |
| *Citrusx paradisi* | | | |
| *Triphasia trifolia* | | | |
| *Vitis vinifera* | | | |
| *Prunus persica* | | | |
| *Prunus armeriana* | | | |
| *Prunus cerasus* | | | |
| *Actinidia chinensis* | | | |
| *Zea mais* | | | |
| *Pisum sativum* | | | |
| *Phaseolus vulgaris* | | | |
| *Sorghum halepense* | | | |
| *Cynara cardunculus* | | | |
| *Arctium lappa* | | | |
| *Lactuca sativa* | | | |
| *Helianthus annuus* | | | |
| *Ficus carica* | | | |
| *Humulus lupulus* | | | |
| *Avena sativa* | | | |
| *Nasturtium officinale* | | | |
| *Armoracia rusticana* | | | |
| *Hordeum vulgare* | | | |
| *Anthriscus cerefolium* | | | |
| *Allium cepa* | | | |
| *Allium porum* | | | |
| *Carum petroselinum* | | | |
| *Solanum tuberosum* | | | |
| *Solanum lycopersicum* | | | |
| *Solanum melongena* | | | |
| *Raphanus sativus* | | | |
| *Brassica oleracea capitata* | | | |
| *Brassica rapa rapa* | | | |
| *Brassica nigra* | | | |
| *Olea europaea* | | | |
| *Urtica dioica* | | | |
| *Rumex acetosa* | | | |

La région amplifiée montre non seulement une variation de taille entre les différentes espèces, mais aussi une variation de séquences. Il est intéressant de remarquer que le niveau de variabilité permet d'identifier la grande majorité des espèces consommées. Cependant, les espèces proches peuvent dans certains cas ne pas être discernables. C'est le cas dans notre exemple entre le blé et le seigle, et entre le chou et le navet.

Le tableau 3 représente les noms communs et origines des séquences des aliments du tableau 2. LECA = séquences réalisées par les inventeurs.

**Tableau 3**

| **Nom scientifique** | **Nom de l'aliment** | **Origine** |
|---|---|---|
| *Theobroa cacao* | Cacao | LECA |
| *Beta vulgaris* | Bettrave sucrière | LECA |
| *Castanea sativa* | Châtaigne | GenBank: AF133653 |
| *Cannabis sativa* | Cannabis | GenBank: AF501598 |
| *Cicer arietinum* | Pois chiche | GenBank: AB 117648 |
| *Saccharum officinarum* | Canne à sucre | GenBank: AY116253 |
| *Asparagus officinalis* | Asperge | GenBank: AJ441164 |
| *Triticum aestivum* | Blé | GenBank: AB042240 |
| *Secale cereale* | Seigle | GenBank: AF519162 |
| *Oryza sativa* | Riz | GenBank: X15901 |
| *Panicum miliaceum* | Millet | GenBank: AY142738 |
| *Ribes aureum* | Groseiller à fleur jaune | GenBank: AF374816 |
| *Fragaria vesca* | Fraise | LECA |
| *Citrus x paradisi* | Citron/Orange | GenBank: AY295277 |
| *Triphasia trifolia* | Poncirus | GenBank: AY295297 |
| *Vitis vinifera* | Raisin | LECA |
| *Prunus persica* | Pêche | GenBank: AF348560 |
| *Prunus armeriana* | Abricot | LECA |
| *Prunus cerasus* | Cerise | LECA |
| *Actinidia chinensis* | Kiwi | GenBank: AF534655 |
| *Zea mais* | Maïs | GenBank: NC 001666 |
| *Pisum sativum* | Petit pois | LECA |
| *Phaseolus vulgaris* | Haricot | GenBank: AY077945 |
| *Sorghum halepense* | Sorgho | GenBank: AY116244 |
| *Cynara cardunculus* | Artichaud | GenBank: AF129828 |
| *Arctium lappa* | Grande bardane | GenBank: AF129824 |
| *Lactuca sativa* | Laitue | GenBank: U82042 |
| *Helianthus annuus* | Tournesol | GenBank: U82038 |
| *Ficus carica* | Figue | LECA |
| *Humulus lupulus* | Houblon | GenBank: AF501599 |
| *Avena sativa* | Avoine | GenBank: X75695 |
| *Nasturtium officinale* | Cresson | GenBank: AY122457 |
| *Armoracia rusticana* | Raifort | GenBank: AF079350 |
| *Hordeum vulgare* | Orge | GenBank: X74574 |
| *Anthriscus cerefolium* | Cerfeuil | GenBank: AF432022 |
| *Allium cepa* | Oignon | LECA |
| *Allium porum -* | Poireau | LECA |
| *Carum petroselinum* | Persil | LECA |
| *Solanum tuberosum* | Pomme de terre | LECA |
| *Solanum lycopersicum* | Tomate | GenBank: AY098703 |
| *Solanum melongena* | Aubergine | GenBank: AY266240 |
| *Raphanus sativus* | Radis | GenBank: AF451576 |
| *Brassica oleracea capitata* | Choux | GenBank: AF451574 |
| *Brassica rapa rapa* | Navet | GenBank: AF451573 |
| *Brassica nigra* | Moutarde noire | GenBank: AF451579 |
| *Olea europaea* | Olive | LECA |
| *Urtica dioica* | Ortie | GenBank: AY208725 |
| *Rumex acetosa* | Oseille | GenBank: AY177334 |

### 4) Exemples d'applications à des substrats dégradés

Nous avons réalisé plusieurs expérimentations qui démontrent clairement la validité de l'approche proposée dans la présente demande.

L'ADN de plusieurs substrats complexes et/ou transformés a été extrait en utilisant un kit d'extraction classique et en suivant les instructions du fabriquant (Dneasy Plant Mini kit, Qiagen). Les substrats testés sont les suivants :
(i) Sucre de canne
(ii) Pomme de terre cuite
(iii) Pâte cuite
(iv) Potage en sachet lyophilisé

Pour les aliments solides, l'ADN a été extrait à partir de 50 mg de poids sec. Le volume final de l'extrait d'ADN a été récupéré dans 200 µl.

L'amplification a été réalisée en utilisant les amorces g et h (Tableau 1), et les conditions d'amplification suivantes :
(i) volume final : 25µl
(ii) MgCl₂: 2 mM
(iii) dNTP : 0,2 mM chacun
(iv) Amorces : 1 µM chacune
(v) Taq polymérase (Amplitaq Gold, Perkin Elmer) : 1 unité
(vi) BSA : 0,2 µl par tube
(vii) Volume d'extrait d'ADN utilisé : 2,5 µl (1/80 de l'extrait)
(viii) Dénaturation initiale de 10 mn à 95°C
(ix) Nombre de cycles : 35 (sauf pour le sucre de canne : 50)
(x) Dénaturation : 30 s à 95°C, Hybridation : 30 s à 55°C, Elongation : 60 s à 72°C

La Figure 2 illustre un résultat d'amplification. Les produits d'amplification ont ensuite été séquencés par séquençage direct sur séquenceur automatique capillaire ABI 3100. Les séquences obtenues pour le sucre de canne, la pomme de terre cuite, et la pâte cuite sont identiques respectivement aux séquences de la canne à sucre (*Saccharum offcinarum*), de la pomme de terre (*Solanum tuberosum*), et du blé (*Triticum aestivum*). En revanche, la séquence obtenue par séquençage direct pour le potage en sachet lyophilisé n'est pas lisible, ce qui indique qu'il s'agit d'un mélange. Nous avons donc cloné le produit PCR du potage en sachet lyophilisé afin de séparer les différentes molécules. Sur 23 clones séquencés, nous avons obtenu 19 clones contenant la séquence du poireau, trois clones contenant la séquence de la pomme de terre, et un seul clone contenant la séquence de l'oignon.

Le tableau 4 montre les résultats du clonage du produit d'amplification obtenu à partir du potage en sachet lyophilisé (23 clones séquencés). Les résultats obtenus correspondent à la composition indiquée sur le sachet.

**Tableau 4**

| **Séquence obtenue** | **Identification** | **nombre** |
|---|---|---|
| | Poireau | 9 |
| | Pomme de terre | 3 |
| | Oignon | 1 |

### 5) Exemple comparatif sur substrat déqradé avec les amorces g, h et c, d

L'objectif de cette expérimentation était de comparer la présente invention avec l'approche publiée en 1991 (Taberlet P, Gielly L, Pautou G, Bouvet J (1991) Universal primers for amplification of three non-coding regions of chloroplast DNA. Plant Molecular Biology, 17, 1105-1109).

L'ADN génomique a été extrait à partir de 100 mg de mie de pain en utilisant un kit d'extraction (Dneasy Plant Mini kit, Qiagen) et en suivant les instructions du fournisseur. Le volume final de l'extrait d'ADN a été récupéré dans 100 µl.

L'amplification a été réalisée en utilisant d'une part les amorces g et h, et d'autre part les amorces c et d (Taberlet P, Gielly L, Pautou G, Bouvet J (1991) Universal primers for amplification of three non-coding regions of chloroplast DNA. Plant Molecular Biology, 17, 1105-1109). Les conditions d'amplifications suivantes ont été appliquées :
volume final : 25µl
MgCl2: 2 mM
dNTP : 0,2 mM chacun
Amorces : 1 µM chacune
Taq polymérase (Amplitaq Gold, Perkin Elmer) : 1 unité
BSA : 0,2 µl par tube
Volume d'extrait d'ADN utilisé : 2,5 µl (1/80 de l'extrait)
Dénaturation initiale de 10 mn à 95°C
Nombre de cycles : 25
Dénaturation : 30 s à 95°C, Hybridation : 30 s à 55°C, Elongation : 60 s à 72°C

La Figure 3 présente les résultats obtenus. Aucun produit d'amplification n'apparaît avec les amorces c et d, alors qu'un produit d'amplification est obtenu avec les amorces g et h.

## Revendications

1. Paire d'oligonucléotides **caractérisée en ce que** le premier oligonucléotide s'hybride à la séquence de la SEQ ID No. 68 et le deuxième oligonucléotide s'hybride à la séquence de la SEQ ID No. 69 dans des conditions de stringence suffisantes pour l'amplification sélective d'une région variable de l'intron du gène chloroplastique *trn*L du tabac dont la séquence est représentée à la SEQ ID No. 3.

2. Paire d'oligonucléotides **caractérisée en ce que** le premier oligonucléotide s'hybride à la séquence de la SEQ ID No. 68 et le deuxième oligonucléotide s'hybride à la séquence de la SEQ ID No. 69 dans des conditions de stringence suffisantes pour l'amplification sélective d'une région variable de l'intron du gène chloroplastique *trn*L des végétaux dont la séquence est représentée aux SEQ ID Nos. 24-67.

3. Paire d'oligonucléotides selon la revendication 1 ou 2 **caractérisée en ce que** l'hybridation s'effectue à 55°C dans un tampon d'amplification comprenant du MgCl₂ 2mM.

4. Paire d'oligonucléotides selon l'une des revendications précédentes **caractérisée en ce que** le premier oligonucléotide est choisi dans le groupe comprenant les SEQ ID Nos. 1, 4-15 et le deuxième oligonucléotide est choisi dans le groupe comprenant les SEQ ID Nos. 2, 16-23.

5. Oligonucléotide **caractérisé en ce que** sa séquence est choisie dans le groupe comprenant la SEQ ID No. 1, la SEQ ID No. 2, les SEQ ID Nos. 4-15 et les SEQ ID Nos. 16-23.

6. Polynucléotide **caractérisé en ce que** sa séquence est choisie dans le groupe comprenant les SEQ ID Nos. 24-67.

7. Paire d'oligonucléotides selon l'une des revendications 1-4, oligonucléotide selon la revendication 5 ou polynucléotide selon la revendication 6 **caractérisés en ce qu'**ils sont immobilisés sur un support solide.

8. Procédé d'amplification d'une région variable de l'ADN chloroplastique des végétaux **caractérisé en ce qu'**il comprend les étapes suivantes :
a) on dispose d'un échantillon comprenant de l'ADN génomique végétal ;
b) on amplifie une région variable de l'ADN chloroplastique avec une paire d'oligonucléotides selon l'une des revendications 1-4 et 7, ou avec au moins un oligonucléotide selon la revendication 5.

9. Procédé d'amplification d'une région variable de l'ADN chloroplastique des végétaux selon la revendication 8 **caractérisé en ce que** à l'étape b) la région variable de l'ADN chloroplastique amplifiée est un polynucléotide dont la séquence est choisie dans le groupe comprenant les SEQ ID Nos. 24-67.

10. Procédé de détection d'une espèce végétale dans un échantillon **caractérisé en ce qu'**il comprend les étapes suivantes :
a) on dispose d'un échantillon suspecté de contenir une espèce végétale ;
b) on effectue une réaction d'amplification avec une paire d'oligonucléotides selon l'une des revendications 1-4 et 7, ou avec au moins un oligonucléotide selon la revendication 5 ;
c) on détecte l'obtention d'un produit d'amplification attestant de la présence d'une espèce végétale dans l'échantillon.

11. Procédé de détection d'une espèce végétale dans un échantillon selon la revendication 10 **caractérisé en ce que** à l'étape c) le produit d'amplification est un polynucléotide dont la séquence est choisie dans le groupe comprenant les SEQ ID Nos. 24-67.

12. Procédé d'identification d'une espèce végétale dans un échantillon **caractérisé en ce qu'**il comprend les étapes suivantes :
a) on dispose d'un échantillon suspecté de contenir une espèce végétale ;
b) on effectue une réaction d'amplification avec une paire d'oligonucléotides selon l'une des revendications 1-4 et 7, ou avec au moins un oligonucléotide selon la revendication 5 ;
c) on analyse le produit d'amplification obtenu pour identifier l'espèce végétale contenue dans l'échantillon.

13. Procédé d'identification d'une espèce végétale dans un échantillon selon la revendication 12 dans lequel à l'étape c) le produit d'amplification est un polynucléotide dont la séquence est choisie dans le groupe comprenant les SEQ ID Nos. 24-67.

14. Procédé d'identification d'une espèce végétale dans un échantillon selon la revendication 12 ou 13 dans lequel à l'étape c) on détermine la séquence du produit d'amplification pour identifier l'espèce végétale contenue dans l'échantillon.

15. Procédé d'identification d'une espèce végétale dans un échantillon selon la revendication 12 ou 13 dans lequel à l'étape c) on hybride le produit d'amplification avec au moins une séquence végétale de référence pour identifier l'espèce végétale contenue dans l'échantillon.

16. Procédé d'identification d'une espèce végétale dans un échantillon selon la revendication 15 dans lequel la séquence de référence est choisie dans le groupe comprenant les SEQ ID Nos. 3 et 24-67.

17. Procédé d'identification d'une espèce végétale dans un échantillon selon la revendication 12 ou 13 dans lequel à l'étape c) on analyse le produit d'amplification par électrophorèse pour identifier l'espèce végétale contenue dans l'échantillon.

18. Utilisation de la région variable de l'intron du gène chloroplastique *trn*L des végétaux correspondant aux positions 49425 à 49466 de l'ADN chloroplastique du tabac pour la détection et l'identification d'espèces végétales.

19. Utilisation selon la revendication 18 dans laquelle la région variable de l'intron du gène chloroplastique *trn*L des végétaux est un polynucléotide dont la séquence est choisie dans le groupe comprenant les SEQ ID Nos. 24-67.

## Claims

1. Pair of oligonucleotides **characterized in that** the first oligonucleotide hybridizes to the SEQ ID No. 68 sequence and **in that** the second oligonucleotide hybridizes to the SEQ ID No. 69 sequence under stringency conditions which are sufficient for the selective amplification of a variable region of the intron of the *trn*L chloroplast gene of tobacco, whose sequence is represented at SEQ ID No. 3.

2. Pair of oligonucleotides **characterized in that** the first oligonucleotide hybridizes to the SEQ ID No. 68 sequence and **in that** the second oligonucleotide hybridizes to the SEQ ID No. 69 sequence under stringency conditions which are sufficient for the selective amplification of a variable region of the intron of the *trn*L chloroplast gene of plants whose sequence is represented at SEQ ID Nos. 24-67.

3. Pair of oligonucleotides according to Claim 1 or 2, **characterized in that** hybridization occurs at 55°C in an amplification buffer comprising 2mM MgCl₂.

4. Pair of oligonucleotides according to one of the preceding claims, **characterized in that** the first oligonucleotide is chosen from the group comprising SEQ ID Nos. 1, 4-15 and **in that** the second oligonucleotide is chosen from the group comprising SEQ ID Nos. 2, 16-23.

5. Oligonucleotide **characterized in that** its sequence is chosen from the group comprising SEQ ID No. 1, SEQ ID No. 2, SEQ ID Nos. 4-15, SEQ ID Nos. 16-23.

6. Polynucleotide **characterized in that** its sequence is chosen from the group comprising SEQ ID Nos. 24-67.

7. Pair of oligonucleotides according to one of Claims 1-4, oligonucleotide according to Claim 5 or polynucleotide according to Claim 6, **characterized in that** they are immobilized on a solid support.

8. Method for amplifying a variable region of chloroplast DNA of plants, **characterized in that** it comprises the following steps:
a) a sample including plant genomic DNA is provided;
b) a variable region of chloroplast DNA is amplified with a pair of oligonucleotides according to one of Claims 1-4 and 7, or with at least one oligonucleotide according to Claim 5.

9. Method for amplifying a variable region of chloroplast DNA of plants according to Claim 8, **characterized in that,** at step b), the variable region of amplified chloroplast DNA is a polynucleotide whose sequence is chosen from the group comprising SEQ ID Nos. 24-67.

10. Method for detecting a plant species in a sample, **characterized in that** it comprises the following steps:
a) a sample suspected of containing a plant species is provided;
b) an amplification reaction is carried out with a pair of oligonucleotides according to one of Claims 1-4 and 7, or with at least one oligonucleotide according to Claim 5;
c) detection of whether an amplification product proving the presence of a plant species in the sample is obtained.

11. Method for detecting a plant species in a sample according to Claim 10, **characterized in that,** at step c), the amplification product is a polynucleotide whose sequence is chosen from the group comprising SEQ ID Nos. 24-67.

12. Method for identifying a plant species in a sample, **characterized in that** it comprises the following steps:
a) a sample suspected of containing a plant species is provided;
b) an amplification reaction is carried out with a pair of oligonucleotides according to one of Claims 1-4 and 7, or with at least one oligonucleotide according to Claim 5;
c) the amplification product thus obtained is analyzed to identify the plant species contained in the sample.

13. Method for identifying a plant species in a sample according to Claim 12, in which, at step c), the amplification product is a polynucleotide whose sequence is chosen from the group comprising SEQ ID Nos. 24-67.

14. Method for identifying a plant species in a sample according to Claim 12 or 13, in which, at step c), the sequence of the amplification product is determined for identifying the plant species contained in the sample.

15. Method for identifying a plant species in a sample according to Claim 12 or 13, in which, at step c), the amplification product is hybridized with at least one reference plant sequence for identifying the plant species contained in the sample.

16. Method for identifying a plant species in a sample according to Claim 15, in which the reference sequence is chosen from the group comprising SEQ ID Nos. 3 and 24-67.

17. Method for identifying a plant species in a sample according to Claim 12 or 13, in which, at step c), the amplification product is analyzed by electrophoresis for identifying the plant species contained in the sample.

18. Use of the variable region of the intron of the *trn*L chloroplast gene of plants corresponding to positions 49425 to 49466 of the chloroplast DNA of tobacco for detecting and identifying plant species.

19. Use according to Claim 18 in which the variable region of the intron of the *trn*L chloroplast gene of plants is a polynucleotide whose sequence is chosen from the group comprising SEQ ID Nos. 24-67.

## Patentansprüche

1. Oligonukleotidpaar, **dadurch gekennzeichnet, dass** das erste Oligonukleotid mit der Sequenz SEQ ID NR: 68 hybridisiert und das zweite Oligonukleotid mit der Sequenz SEQ ID NR: 69 hybridisiert, jeweils unter Bedingungen einer ausreichenden Stringenz für eine selektive Amplifikation einer variablen Region des Introns des Chloroplastengens *trn*L von Tabak, dessen Sequenz in SEQ ID NR: 3 angegeben ist.

2. Oligonukleotidpaar, **dadurch gekennzeichnet, dass** das erste Oligonukleotid mit der Sequenz SEQ ID NR: 68 hybridisiert und das zweite Oligonukleotid mit der Sequenz SEQ ID NR: 69 hybridisiert, jeweils unter Bedingungen einer ausreichenden Stringenz für eine selektive Amplifikation einer variablen Region des Introns des Chloroplastengens *trn*L von Pflanzen, deren Sequenz in den SEQ ID NR: 24-67 angegeben ist.

3. Oligonukleotidpaar nach Anspruch 1 oder 2, **dadurch gekennzeichnet, dass** die Hybridisierung bei 55°C in einem Amplifikationspuffer, der 2 mM MgCl₂ enthält, erfolgt.

4. Oligonukleotidpaar nach einem der vorhergehenden Ansprüche, **dadurch gekennzeichnet, dass** das erste Oligonukleotid aus der Gruppe ausgewählt ist, die die SEQ ID NR: 1, 4-15 umfasst, und das zweite Oligonukleotid aus der Gruppe ausgewählt ist, die die SEQ ID NR: 2, 16-23 umfasst.

5. Oligonukleotid, **dadurch gekennzeichnet, dass** seine Sequenz aus der Gruppe ausgewählt ist, die die SEQ ID NR: 1, SEQ ID NR: 2, SEQ ID NR: 4-15 und die SEQ ID NR: 16-23 umfasst.

6. Polynukleotid, **dadurch gekennzeichnet, dass** seine Sequenz aus der Gruppe ausgewählt ist, die die SEQ ID NR: 24-67 umfasst.

7. Oligonukleotidpaar nach einem der Ansprüche 1-4, Oligonukleotid nach Anspruch 5 oder Polynukleotid nach Anspruch 6, **dadurch gekennzeichnet, dass** sie auf einem festen Träger immobilisiert sind.

8. Verfahren zur Amplifikation einer variablen Region von Chloroplasten-DNA von Pflanzen, wobei man in den folgenden Schritten:
a) über eine Probe verfügt, die pflanzliche genomische DNA umfasst;
b) eine variable Region von Chloroplasten-DNA mit einem Oligonukleotidpaar nach einem der Ansprüche 1-4 und 7 oder mit mindestens einem Oligonukleotid nach Anspruch 5 amplifiziert.

9. Verfahren zur Amplifikation einer variablen Region von Chloroplasten-DNA von Pflanzen nach Anspruch 8, **dadurch gekennzeichnet, dass** im Schritt b) die amplifizierte variable Region von Chloroplasten-DNA ein Polynukleotid ist, dessen Sequenz aus der Gruppe ausgewählt ist, die die SEQ ID NR: 24-67 umfasst.

10. Verfahren zum Nachweis einer Pflanzenspezies in einer Probe, **dadurch gekennzeichnet, dass** man in den folgenden Schritten:
a) über eine Probe verfügt, von der vermutet wird, dass sie eine Pflanzenspezies enthält;
b) eine Amplifikationsreaktion mit einem Oligonukleotidpaar nach einem der Ansprüche 1-4 und 7 oder mit mindestens einem Oligonukleotid nach Anspruch 5 durchführt;
c) das Erhalten eines Amplifikationsprodukts nachweist, das das Vorliegen einer Pflanzenspezies in der Probe bestätigt.

11. Verfahren zum Nachweis einer Pflanzenspezies in einer Probe nach Anspruch 10, **dadurch gekennzeichnet, dass** im Schritt c) das Amplifikationsprodukt ein Polynukleotid ist, dessen Sequenz aus der Gruppe ausgewählt ist, die die SEQ ID NR: 24-67 umfasst.

12. Verfahren zur Identifikation einer Pflanzenspezies in einer Probe, **dadurch gekennzeichnet, dass** man in den folgenden Schritten:
a) über eine Probe verfügt, von der vermutet wird, dass sie eine Pflanzenspezies enthält;
b) eine Amplifikationsreaktion mit einem Oligonukleotidpaar nach einem der Ansprüche 1-4 und 7 oder mit mindestens einem Oligonukleotid nach Anspruch 5 durchführt;
c) das erhaltene Amplifikationsprodukt analysiert, um die in der Probe enthaltene Pflanzenspezies zu identifizieren.

13. Verfahren zur Identifikation einer Pflanzenspezies in einer Probe nach Anspruch 12, wobei im Schritt c) das Amplifikationsprodukt ein Polynukleotid ist, dessen Sequenz aus der Gruppe ausgewählt ist, die die SEQ ID NR: 24-67 umfasst.

14. Verfahren zur Identifikation einer Pflanzenspezies in einer Probe nach Anspruch 12 oder 13, wobei man im Schritt c) die Sequenz des Amplifikationsprodukts bestimmt, um die in der Probe enthaltene Pflanzenspezies zu identifizieren.

15. Verfahren zur Identifikation einer Pflanzenspezies in einer Probe nach Anspruch 12 oder 13, wobei man im Schritt c) das Amplifikationsprodukt mit mindestens einer pflanzlichen Referenzsequenz hybridisiert, um die in der Probe enthaltene Pflanzenspezies zu identifizieren.

16. Verfahren zur Identifikation einer Pflanzenspezies in einer Probe nach Anspruch 15, wobei die Referenzsequenz aus der Gruppe ausgewählt ist, die die SEQ ID NR: 3 und 24-67 umfasst.

17. Verfahren zur Identifikation einer Pflanzenspezies in einer Probe nach Anspruch 12 oder 13, wobei man im Schritt c) das Amplifikationsprodukt mittels Elektrophorese analysiert, um die in der Probe enthaltene Pflanzenspezies zu identifizieren.

18. Verwendung der variablen Region des Introns des Chloroplastengens *trn*L von Pflanzen, die den Positionen 49425 bis 49466 der Chloroplasten-DNA von Tabak entspricht, zum Nachweis und zur Identifikation von Pflanzenspezies.

19. Verwendung nach Anspruch 18, wobei die variable Region des Introns des Chloroplastengens *trn*L von Pflanzen ein Polynukleotid ist, dessen Sequenz aus der Gruppe ausgewählt ist, die die SEQ ID NR: 24-67 umfasst.


### SEQUENCE LISTING

<110> Université Joseph Fourier CNRS
<120> Amorces universelles et leur utilisation pour la détection et l'identification de végétaux dans des mélanges complexes
<130> KH/SG/BR051584
<160> 69
<170> PatentIn version 3.2
<210> 1
   <211> 17
   <212> DNA
   <213> Nicotiana tabacum
<400> 1
   gggcaatcct gagccaa 17
<210> 2
   <211> 22
   <212> DNA
   <213> Nicotiana tabacum
<400> 2
   ccattgagtc tctgcaccta tc 22
<210> 3
   <211> 40
   <212> DNA
   <213> Nicotiana tabacum
<400> 3
   atcctgtttt ccgaaaacaa acaaaggttc agaaaaaaag 40
<210> 4
   <211> 17
   <212> DNA
   <213> Prunus persica
<400> 4
   gggcgatcct gagccaa 17
<210> 5
   <211> 17
   <212> DNA
   <213> Urtica dioica
<400> 5
   gggcaatcct gaaccaa 17
<210> 6
   <211> 17
   <212> DNA
   <213> Séquence artificielle
<400> 6
   aggcaatcct gagccaa 17
<210> 7
   <211> 17
   <212> DNA
   <213> Séquence artificielle
<400> 7
   cggcaatcct gagccaa 17
<210> 8
   <211> 17
   <212> DNA
   <213> séquence artificielle
<400> 8
   gggtaatcct gagccaa 17
<210> 9
   <211> 17
   <212> DNA
   <213> séquence artificielle
<400> 9
   gggcgatcct gagccaa 17
<210> 10
   <211> 17
   <212> DNA
   <213> Séquence artificielle
<400> 10
   gggctatcct gagccaa 17
<210> 11
   <211> 17
   <212> DNA
   <213> séquence artificielle
<400> 11
   gggccatcct gagccaa 17
<210> 12
   <211> 17
   <212> DNA
   <213> Séquence artificielle
<400> 12
   gggcaattct gagccaa 17
<210> 13
   <211> 17
   <212> DNA
   <213> Séquence artificielle
<400> 13
   gggcaatcct gggccaa 17
<210> 14
   <211> 17
   <212> DNA
   <213> Séquence artificielle
<400> 14
   gggcaatcct gacccaa 17
<210> 15
   <211> 17
   <212> DNA
   <213> Séquence artificielle
<400> 15
   gggcaatcct gatccaa 17
<210> 16
   <211> 22
   <212> DNA
   <213> Beta vulgaris
<400> 16
   cctttgagtc tctgcaccta tc 22
<210> 17
   <211> 22
   <212> DNA
   <213> Phaseolus vulgaris
<400> 17
   ccatagagtc tctgcaccta tc 22
<210> 18
   <211> 22
   <212> DNA
   <213> Cynara cardunculus
<400> 18
   ccatcgagtc tctgcaccta tc 22
<210> 19
   <211> 22
   <212> DNA
   <213> Urtica dioica
<400> 19
   ccgttgagtc tctgcaccta tc 22
<210> 20
   <211> 22
   <212> DNA
   <213> Séquence artificielle
<400> 20
   ccattgagtc tctgtaccta tc 22
<210> 21
   <211> 22
   <212> DNA
   <213> Séquence artificielle
<400> 21
   ccattgagtc tcggcaccta tc 22
<210> 22
   <211> 22
   <212> DNA
   <213> Séquence artificielle
<400> 22
   ccgtcgagtc tctgcaccta tc 22
<210> 23
   <211> 22
   <212> DNA
   <213> séquence artificielle
<400> 23
   ccattgagtc tctgcacctg tc 22
<210> 24
   <211> 65
   <212> DNA
   <213> Theobroma cacao
<400> 24
   atcctattat tttattattt tacgaaacta aacaaaggtt cagcaagcga gaataataaa 60
   aaaag 65
<210> 25
   <211> 56
   <212> DNA
   <213> Beta vulgaris
<400> 25
   ctcctttttt caaaagaaaa aaaataagga ttccgaaaac aagaataaaa aaaaag 56
<210> 26
   <211> 55
   <212> DNA
   <213> Castanea sativa
<400> 26
   atcctatttt acgaaaacaa ataagggttc agaagaaagc gagaataaaa aaaag 55
<210> 27
   <211> 55
   <212> DNA
   <213> Cannabis sativa
<400> 27
   atccggtttt ctgaaaacaa acaaggattc agaaagcaat aataaaaaag aatag 55
<210> 28
   <211> 54
   <212> DNA
   <213> cicer arietinum
<400> 28
   atcctgcttt cggaaaacaa acaaaaaaag ttcagaaagt taaaatcaaa aaag 54
<210> 29
   <211> 53
   <212> DNA
   <213> Saccharum officinarum
<400> 29
   atcccctttt ttgaaaaaac aagtggttct caaactagaa cccaaaggaa aag 53
<210> 30
   <211> 53
   <212> DNA
   <213> Asparagus officinalis
<400> 30
   atctttatgt ttagaaaaac aagggtttta atttaaaaac tagaagaaaa agg 53
<210> 31
   <211> 52
   <212> DNA
   <213> Triticum aestivum
<400> 31
   atccgtgttt tgagaaaaca aggggttctc gaactagaat acaaaggaaa ag 52
<210> 32
   <211> 52
   <212> DNA
   <213> oryza sativa
<400> 32
   atccatgttt tgagaaaaca agcggttctc gaactagaac ccaaaggaaa ag 52
<210> 33
   <211> 52
   <212> DNA
   <213> Panicum miliaceum
<400> 33
   atcccttttt tgaaaaaaca agtggttctc aaactagaac ccaaaggaaa ag 52
<210> 34
   <211> 52
   <212> DNA
   <213> Ribes aureum
<400> 34
   atcctgtttt acaaacaaaa cacaagagtt cacaaagaga gaataaaaaa ag 52
<210> 35
   <211> 52
   <212> DNA
   <213> Fragaria vesca
<400> 35
   atcccgtttt atgaaaacaa acaagggttt cagaaagcga gaataaataa ag 52
<210> 36
   <211> 52
   <212> DNA
   <213> citrus X paradisi
<400> 36
   atcctcttct cttttccaag aacaaacagg ggttcagaaa gcgaaaaagg gg 52
<210> 37
   <211> 52
   <212> DNA
   <213> Triphasia trifolia
<400> 37
   atcctcttct cttttccaag aacaaacagg ggttcagaaa gcgaaaaagg gg 52
<210> 38
   <211> 51
   <212> DNA
   <213> Vitis vinifera
<400> 38
   atcctgtttt ccgaaaacaa ccaagggttc agaaaacgat aataaaaaaa g 51
<210> 39
   <211> 51
   <212> DNA
   <213> Prunus persica
<400> 39
   atcctgtttt attaaaacaa acaagggttt cataaaccga gaataaaaaa g 51
<210> 40
   <211> 51
   <212> DNA
   <213> Actinidia chinensis
<400> 40
   atcctttttt tcgaaaacaa acaaagattc agaaagcgaa aataaaacaa g 51
<210> 41
   <211> 51
   <212> DNA
   <213> Zea mays
<400> 41
   atcccttttt tgaaaaacaa gtggttctca aactagaacc caaaggaaaa g 51
<210> 42
   <211> 51
   <212> DNA
   <213> Pisum sativum
<400> 42
   atccttcttt ctgaaaacaa ataaaagttc agaaagtgaa aatcaaaaaa g 51
<210> 43
   <211> 50
   <212> DNA
   <213> Phaseolus vulgaris
<400> 43
   atcccgtttt ctgaaaaaaa gaaaaattca gaaagtgata ataaaaaagg 50
<210> 44
   <211> 50
   <212> DNA
   <213> Sorghum halepense
<400> 44
   atccactttt ttcaaaaaag tggttctcaa actagaaccc aaaggaaaag 50
<210> 45
   <211> 50
   <212> DNA
   <213> cynara cardunculus
<400> 45
   atcacgtttt ccgaaactaa acaaaggttc agaaagcgaa aatcaaaaag 50
<210> 46
   <211> 50
   <212> DNA
   <213> Arctium lappa
<400> 46
   atcacgtttt ccgaaaacaa acaaaggttc agaaagcgaa aataaaaaag 50
<210> 47
   <211> 50
   <212> DNA
   <213> Lactuca sativa
<400> 47
   atcacgtttt ccgaaaacaa acaacggttc agaaagcgaa aatcaaaaag 50
<210> 48
   <211> 50
   <212> DNA
   <213> Helianthus annuus
<400> 48
   atcacgtttt ccgaaaacaa acaaaggttc agaaagcgaa aataaaaaag 50
<210> 49
   <211> 50
   <212> DNA
   <213> Ficus carica
<400> 49
   atccggtttt ctgaaaacaa acaagggttc agaaggcgat aataaaaaag 50
<210> 50
   <211> 49
   <212> DNA
   <213> Humulus lupulus
<400> 50
   atccggtttt ctgaaaacaa acaaggattc agaaagcaat aataaaggg 49
<210> 51
   <211> 48
   <212> DNA
   <213> Avena sativa
<400> 51
   atccgtgttt tgagaggggg gttctcgaac tagaatacaa aggaaaag 48
<210> 52
   <211> 48
   <212> DNA
   <213> Nasturtium officcinale
<400> 52
   atccttgttt acgcaaacaa accggagttt agaaagcgag aaaaaagg 48
<210> 53
   <211> 48
   <212> DNA
   <213> Armoracia rusticana
<400> 53
   atccttgttt acgcgaacaa acctgagttt agaaagcgag ataaaagg 48
<210> 54
   <211> 47
   <212> DNA
   <213> Hordeum vulgare
<400> 54
   atccgtgttt tgagaaggga ttctcgaact agaatacaaa ggaaaag 47
<210> 55
   <211> 47
   <212> DNA
   <213> Anthriscus cerefolium
<400> 55
   atcctatttt ttccaaaaac aaacaaaggc ccagaaggtg aaaaaag 47
<210> 56
   <211> 47
   <212> DNA
   <213> Allium cepa
<400> 56
   atctttcttt tttgaaaaac aagggtttaa aaaagagaat aaaaaag 47
<210> 57
   <211> 47
   <212> DNA
   <213> Allium porrum
<400> 57
   atctttattt tttgaaaaac aagggtttaa aaaagagaat aaaaaag 47
<210> 58
   <211> 45
   <212> DNA
   <213> Carum petroselinum
<400> 58
   atcctatttt ccaaaaacaa acaaaggccc agaaggtgaa aaaag 45
<210> 59
   <211> 40
   <212> DNA
   <213> Solanum tuberosum
<400> 59
   atcctgtttt ctgaaaacaa acaaaggttc agaaaaaaag 40
<210> 60
   <211> 40
   <212> DNA
   <213> Solanum lycopersicum
<400> 60
   atcctgtttt ctgaaaacaa accaaggttc agaaaaaaag 40
<210> 61
   <211> 40
   <212> DNA
   <213> Solanum melongena
<400> 61
   atcctgtttt ctcaaaacaa acaaaggttc agaaaaaaag 40
<210> 62
   <211> 39
   <212> DNA
   <213> Raphanus sativus
<400> 62
   atcctgagtt acgcgaacaa accagagttt agaaagcgg 39
<210> 63
   <211> 39
   <212> DNA
   <213> Brassica oleracea capitata
<400> 63
   atcctgggtt acgcgaacaa aacagagttt agaaagcgg 39
<210> 64
   <211> 39
   <212> DNA
   <213> Brassica nigra
<400> 64
   atcctgggtt acgcgaacaa accagagttt agaaagcgg 39
<210> 65
   <211> 39
   <212> DNA
   <213> olea europaea
<400> 65
   atcctgtttt cccaaaacaa aggttcagaa agaaaaaag 39
<210> 66
   <211> 34
   <212> DNA
   <213> Urtica dioica
<400> 66
   atctggtgtt ataaaacaaa gcgataaaaa aaag 34
<210> 67
   <211> 31
   <212> DNA
   <213> Rumex acetosa
<400> 67
   ctcctccttt ccaaaaggaa gaataaaaaa g 31
<210> 68
   <211> 17
   <212> DNA
   <213> Séquence artificielle
<400> 68
   ttggctcagg attgccc 17
<210> 69
   <211> 22
   <212> DNA
   <213> Séquence artificielle
<400> 69
   gataggtgca gagactcaat gg 22